(19) Europäisches Patentamt
European Patent Office
Office européen des brevets

(11) **EP 2 588 093 B1**

(12) **EUROPEAN PATENT SPECIFICATION**

(45) Date of publication and mention
of the grant of the patent:
**15.06.2016 Bulletin 2016/24**

(51) Int Cl.:
***A61K 31/135*** *(2006.01)*    ***A61P 1/00*** *(2006.01)*
***A61K 31/137*** *(2006.01)*

(21) Application number: **11733571.1**

(86) International application number:
**PCT/EP2011/003217**

(22) Date of filing: **29.06.2011**

(87) International publication number:
**WO 2012/000666 (05.01.2012 Gazette 2012/01)**

(54) **TAPENTADOL FOR USE IN THE TREATMENT OF IRRITABLE BOWEL SYNDROME**

TAPENTADOL ZUR VERWENDUNG BEI DER BEHANDLUNG VON REIZDARMSYNDROM

TAPENTADOL DESTINÉ À ÊTRE UTILISÉ DANS LE TRAITEMENT DU SYNDROME DU CÔLON IRRITABLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.06.2010 EP 10006782**

(43) Date of publication of application:
**08.05.2013 Bulletin 2013/19**

(73) Proprietor: **Grünenthal GmbH
52078 Aachen (DE)**

(72) Inventors:
• **SCHIENE, Klaus
41363 Jüchen (DE)**
• **BLOMS-FUNKE, Petra
52146 Würselen (DE)**
• **CHRISTOPH, Thomas
52080 Aachen (DE)**
• **SCHRÖDER, Wolfgang
52074 Aachen (DE)**

(74) Representative: **Brosch, Oliver et al
Kutzenberger Wolff & Partner
Theodor-Heuss-Ring 23
50668 Köln (DE)**

(56) References cited:
**EP-A1- 1 985 292      EP-A1- 1 997 493
WO-A2-2009/094563**

## Description

The invention relates to tapentadol for use in the treatment of irritable bowel syndrome.

Irritable bowel syndrome (IBS or spastic colon) is characterized by abdominal pain and/or discomfort related to abnormal bowel habits. It is probably the most common disorder encountered by gastroenterologists and also the most common gastrointestinal disorder seen in primary care. In the Western world, IBS appears to affect up to 20 % of the population at any given time, although the prevalence figures vary substantially depending on the definition of IBS (Posserud I, Ersryd A, Simrén M, Functional findings in irritable bowel syndrome. World J. Gastroenterol. 2006; 12(18):2830-2838). Due to its high prevalence and, for many patients, chronic nature and incapacitating symptoms the cost of IBS to society is substantial. The pathophysiology of IBS is complex and still incompletely known. Both central and peripheral factors, including psychosocial factors, abnormal gastrointestinal (GI) motility and secretion, visceral hypersensitivity and referred pain, are thought to contribute to the symptoms of IBS. Validated schemata for irritable bowel syndrome are available such as the Manning criteria and the Rome criteria that allow for the diagnosis of irritable bowel syndrome to be made based upon the history of the patient. The subclassification of IBS is based on the predominant symptom of diarrhea (IBS with predominant diarrhea, IBS-D), constipation (IBS with predominant constipation, IBS-C) or mixed symptoms (IBS with alternating constipation and diarrhea, IBS-C)(Grundmann O, Yoon SL, Irritable bowel syndrome: epidemiology, diagnosis and treatment: an update for health-care practitioners; J. Gastroenterol. Hepatol., 2010; 25(4):691-699). Due to the limited efficacy and tolerability of current treatment, there is still a great need to find new treatment alternatives for this big patients group.

A crystalline form of a (3S)-aminomethyl-5-hexanoic acid prodrug and methods of preparing a crystalline form of a (3S)-aminomethyl-5-hexanoic acid prodrug, and methods of using a crystalline form of a (3S)-aminomethyl-5-hexanoic acid prodrug are provided in WO 2009/094563 A2.

EP 1 997 493 A1 refers to a combination of a 5HT7 receptor ligand and an opioid receptor ligand, especially of a 5HT7 receptor agonist and an opioid receptor agonist, a medicament comprising this combination, or the use of this combination for the treatment of the symptoms of pain, the prevention or the prophylaxis of the symptoms of pain.

EP 1 985 292 A1 relates to the use of tapentadol for the manufacture of a medicament comprising at least one administration unit **A** containing dose **a** of tapentadol and at least one administration unit **B** containing dose **b** of tapentadol, where dose **a** < dose **b,** for the treatment of pain.

It was an object of the invention to provide a compound and a medicament for use in the treatment of irritable bowel syndrome, which preferably has advantages over other drugs known from the prior art.

This object is achieved by the subject matter of the claims.

The invention relates to tapentadol for use in the treatment of irritable bowel syndrome. Preferably the disorder to be treated is selected from the group consisting of irritable bowel syndrome with diarrhoea, diarrhea-predominant irritable bowel syndrome, irritable bowel syndrome without diarrhoea, constipation-predominant irritable bowel syndrome, irritable bowel syndrome with alternating stool pattern (irritable bowel syndrome with alternating constipation and diarrhea, mixed irritable bowel syndrome) and post infectious irritable bowel syndrome.

Tapentadol, i.e. (1R,2R)-3-(3-dimethylamino-1-ethyl-2-methyl-propyl)-phenol (CAS no. 175591-23-8), is a synthetic, centrally acting analgesic which is effective in the treatment of moderate to severe, acute or chronic pain.

Tapentadol exhibits a dual mechanism of action, on the one hand as a $\mu$-opioid receptor agonist and on the other as a noradrenaline transporter inhibitor. In humans, the affinity of tapentadol to the recombinantly produced $\mu$-opioid receptor is 18-times less than that of morphine. However, clinical studies have shown the pain-alleviating action of tapentadol to be only two to three times less than that of morphine. The only slightly reduced analgesic efficacy with a simultaneously 18-times reduced affinity to the recombinant $\mu$-opioid receptor indicates that the noradrenaline transporter inhibiting property of tapentadol also contributes to its analgesic efficacy. Consequently, it may be assumed that tapentadol has a similar analgesic efficacy to that of pure $\mu$-opioid receptor agonists but has fewer of the side effects associated with the $\mu$-opioid receptor. The compound can be used in the form of its free base or as a salt or solvate. The production of the free base is known for example from EP-A 693 475.

For the purposes of the present invention tapentadol includes (1R,2R)-3-(3-dimethylamino-1-ethyl-2-methyl-propyl)-phenol as well as physiologically acceptable salts and solvates thereof, in particular (1R,2R)-3-(3-dimethylamino-1-ethyl-2-methyl-propyl)-phenol hydrochloride.

Suitable physiologically acceptable salts include salts of inorganic acids, such as e.g. hydrogen chloride, hydrogen bromide and sulfuric acid, and salts of organic acids, such as methanesulfonic acid, fumaric acid, maleic acid, acetic acid, oxalic acid, succinic acid, malic acid, tartaric acid, mandelic acid, lactic acid, citric acid, glutaminic acid, acetylsalicylic acid, nicotinic acid, aminobenzoic acid, $\alpha$-lipoic acid, hippuric acid and aspartic acid. Tapentadol can also be present as any mixture of the salts of the above-mentioned organic and inorganic acids. The most preferred salt is hydrochloride.

In a preferred embodiment, tapentadol is present in a medicament.

In yet another preferred embodiment, the medicament is a solid medicinal form. Liquid or pasty medicinal forms are also possible.

Preferably, the medicament is formulated for oral administration. However, pharmaceutical forms that are adapted for other administration routes are also possible, for example buccal, sublingual, transmucosal, rectal, intralumbar, intraperitoneal, transdermal, intravenous, intramuscular, intragluteal, intracutaneous and subcutaneous administration.

Depending upon the formulation, the medicament preferably contains suitable additives and/or excipients. Suitable additives and/or excipients for the purpose of the invention are all substances for achieving galenic formulations known to the person skilled in the art from the prior art. The selection of these excipients and the amounts to use depend upon how the medicinal product is to be administered, i.e. orally, intravenously, intraperitoneally, intradermally, intramusuclarly, intranasally, buccally or topically.

Suitable for oral administration are preparations in the form of tablets, chewable tablets, dragees, capsules, granules, drops, juices or syrups; suitable for parenteral, topical and inhalative administration are solutions, suspensions, easily reconstituted dry preparations and sprays. A further possibility is suppositories for use in the rectum. Use in a depot in dissolved form, a carrier foil or a plaster, optionally with the addition of means to encourage penetration of the skin, are examples of suitable percutaneous administration forms.

Examples of excipients and additives for oral administration forms are disintegrants, lubricants, binders, fillers, mould release agents, optionally solvents, flavourings, sugar, in particular carriers, diluents, colorants, antioxidants, etc.

For suppositories, it is possible to use inter alia waxes or fatty acid esters and for parenteral means of application, carriers, preservatives, suspension aids, etc.

Excipients can be for example: water, ethanol, 2-propanol, glycerin, ethylene glycol, propylene glycol, polyethylene glycol, polypropylene glycol, glucose, fructose, lactose, sucrose, dextrose, molasses, starch, modified starch, gelatin, sorbitol, inositol, mannitol, microcrystalline cellulose, methyl cellulose, carboxymethylcellulose, cellulose acetate, shellac, cetyl alcohol, polyvinylpyrrolidone, paraffins, waxes, natural and synthetic rubbers, acacia gum, alginates, dextran, saturated and unsaturated fatty acids, stearic acid, magnesium stearate, zinc stearate, glyceryl stearate, sodium lauryl sulfate, edible oils, sesame oil, coconut oil, groundnut oil, soybean oil, lecithin, sodium lactate, polyoxyethylene and propylene fatty acid ester, sorbitan fatty acid esters, sorbic acid, benzoic acid, citric acid, ascorbic acid, tannic acid, sodium chloride, potassium chloride, magnesium chloride, calcium chloride, magnesium oxide, zinc oxide, silicon dioxide, titanium oxide, titanium dioxide, magnesium sulfate, zinc sulfate, calcium sulfate, potash, calcium phosphate, dicalcium phosphate, potassium bromide, potassium iodide, talc kaolin, pectin, crospovidone, agar and bentonite.

The production of a medicinal product, a medicament and a pharmaceutical composition according to the present invention can be performed with the aid of means, devices, methods and processes which are well known in the prior art of pharmaceutical formulation, such as those described for example in "Remington's Pharmaceutical Sciences", ed AR Gennaro, 17th edition, Mack Publishing Company, Easton, pa. (1985), in particular in Part 8, Chapters 76 to 93.

For example, for a solid formulation, such as a tablet, the active substance of the medicament can be granulated with a pharmaceutical carrier, e.g. conventional tablet ingredients, such as maize starch, lactose, sucrose, sorbitol, talc, magnesium stearate, dicalcium phosphate or physiologically acceptable rubbers, and pharmaceutical diluents, such as water, for example, to form a solid composition containing the active substance in a homogeneous distribution. Here, a homogeneous distribution should be understood as meaning that the active substance is distributed uniformly throughout the entire composition so that this can be easily divided into equally effective single dose forms, such as tablets, capsules, dragees. The solid composition is then divided into single dose forms. The tablets or pills can also be coated or compounded in some other way in order to produce a dosage form with delayed release. Suitable coating means are inter alia polymers acids and mixtures of polymeric acids with materials such as shellac, for example, cetyl alcohol and/or cellulose acetate.

In a preferred embodiment of the present invention tapentadol is present in the medicament in immediate release form. Such medicaments may be particularly useful for treating acute gastrointestinal cramps.

In another preferred embodiment of the present invention tapentadol is present in the medicament in controlled-release form. Such medicaments may be particularly useful for treating chronic conditions.

The term controlled release as used herein refers to any type of release other than immediate release such as delayed release, prolonged release, sustained release, slow release, extended release and the like. These terms are well known to any person skilled in the art as are the means, devices, methods and processes for obtaining such type of release.

Controlled release of tapentadol is possible from formulations such as those for oral, rectal or percutaneous administration. Preferably, the medicament is formulated for once-daily administration, for twice-daily administration (*bid*) or for thrice-daily administration, with twice-daily administration (*bid*) being particularly preferred.

The controlled release of tapentadol can, for example, be achieved by retardation by means of a matrix, a coating or release systems with an osmotic action (see e.g. US-A-2005-58706).

The medicament may contain one or more further drugs besides tapentadol. Preferably, however, the medicament contains tapentadol as the only drug.

In a preferred embodiment, the medicament may contain a vitamin such as B1, B6, or B12, a probiotic such as Lactobacilli spp, or a prebiotic, or any mixture thereof. Suitable probiotics and prebiotics are disclosed for example in R. Spiller, Review article: probiotics and prebiotics in irritable bowel syndrome, Aliment Pharmacol Ther 28, 385-396.

The amounts of tapentadol to be administered to patients may vary depending upon the weight of the patient, the method of administration and the severity of the disease and/or pain. Tapentadol may be administered in amounts up to its maximum daily dosage, which is known to those skilled in the art. In a preferred embodiment, the medicament contains tapentadol in an amount of 10 to 300 mg, more preferably 20 to 290 mg, even more preferably 30 to 280 mg, most preferably 40 to 260 mg, as an equivalent dose based on the free base.

In a preferred embodiment, the mean serum concentration of tapentadol, following twice-daily administration of the medicament over a period of at least three days, more preferably at least four days and in particular at least five days, is on average at least 5.0 ng/ml, at least 10 ng/ml, at least 15 ng/ml or at least 20 ng/ml, more preferably at least 25 ng/ml or at least 30 ng/ml, even more preferably at least 35 ng/ml or at least 40 ng/ml, most preferably at least 45 ng/ml or at least 50 ng/ml and in particular at least 55 ng/ml or at least 60 ng/ml. This means that tapentadol is administered over a period of at least three days twice daily and then, preferably 2 h after the administration, the serum concentration is measured. The authoritative numerical value is then obtained as the mean value for all the patients investigated.

In a preferred embodiment, the mean serum concentration of tapentadol in at the most 50% of the patient population, which preferably comprises at least 100 patients, more preferably in at the most 40%, even more preferably in at the most 30%, most preferably in at the most 20% and in particular in at the most 10% of the patient population, following twice-daily administration over a period of at least three days, more preferably at least four days and in particular at least five days, is on average less than 5.0 ng/ml, preferably less than 7.5 ng/ml, even more preferably less than 10 ng/ml, most preferably less than 15 ng/ml and in particular less than 20 ng/ml.

In a preferred embodiment, the mean serum concentration of tapentadol in at the most 50% of the patient population, comprising preferably at least 100 patients, more preferably in at the most 40%, even more preferably in at the most 30%, most preferably in at the most 20% and in particular in at the most 10% of the patient population, following twice-daily administration over a period of at least three days, more preferably at least four days and in particular at least five days, is on average more than 300 ng/ml, more preferably more than 275 ng/ml, even more preferably more than 250 ng/ml, most preferably more than 225 ng/ml and in particular more than 200 ng/ml.

Preferably, the mean serum concentration of tapentadol in at least 50% or 55% of the patient population, which preferably comprises at least 100 patients, more preferably in at least 60% or 65%, even more preferably in at least 70% or 75%, most preferably in at least 80% or 85% and in particular in at least 90% or 95% of the patient population, following twice-daily administration over a period of at least three days, more preferably at least four days and in particular at least five days, is on average in the range of from 1.0 ng/ml to 500 ng/ml, more preferably in the range of from 2.0 ng/ml to 450 ng/ml, even more preferably in the range of from 3.0 ng/ml to 400 ng/ml, most preferably in the range of from 4.0 ng/ml to 350 ng/ml and in particular in the range of from 5.0 ng/ml to 300 ng/ml.

In a preferred embodiment, the percentage standard deviation (coefficient of variation) of the mean serum concentration of tapentadol, preferably in a patient population of 100 patients, following twice-daily administration of the medicament over a period of at least three days, more preferably at least four days and in particular at least five days, is at the most $\pm$ 90%, more preferably at the most $\pm$ 70%, even more preferably at the most $\pm$ 50%, at the most $\pm$ 45% or at the most $\pm$ 40%, most preferably at the most $\pm$ 35%, at the most $\pm$ 30% or at the most $\pm$ 25% and in particular at the most $\pm$ 20%, at the most $\pm$ 15% or at the most $\pm$ 10%.

Preferably, the serum concentrations are average values, produced from measurements on a patient population of preferably at least 10, more preferably at least 25, even more preferably at least 50, even more preferably at least 75, most preferably at least 100 and in particular at least 250 patients. A person skilled in the art knows how to determine the serum concentrations of tapentadol. In this context, reference is made, for example, to TM Tzschentke et al, Drugs of the Future, 2006, 31(12), 1053.

In a preferred embodiment the medicament

- is formulated for oral administration;
- is a solid and/or pressed and/or film-coated medicinal form; and/or
- contains tapentadol having a controlled release from a matrix; and/or
- contains tapentadol in a amount of 0.001 to 99.999 % by weight, more preferably 0.1 to 99.9 % by weight, even more preferably 1.0 to 99.0 % by weight, even more preferably 2.5 to 80 % by weight, most preferably 5.0 to 50 % by weight and in particular 7.5 to 40 % by weight, based on the total weight of the medicament; and/or
- contains a physiologically acceptable carrier and/or physiologically acceptable excipients; and/or
- has a total mass in the range of from 25 to 2,000 mg, more preferably 50 to 1,800 mg, even more preferably 60 to 1,600 mg, even more preferably 70 to 1,400 mg, most preferably 80 to 1,200 mg and in particular 100 to 1,000 mg, and/or

- is selected from the group consisting of tablets, capsules, pellets and granules.

The medicament can be provided as a simple tablet and as a coated tablet (e.g. as a film-coated tablet or dragee). The tablets are usually round and biconvex, but oblong shapes are also possible. Granules, spheroids, pellets or microcapsules, which are used to fill sachets or capsules or pressed into disintegrating tablets, are also possible.

Medicaments containing at least 0.001 to 99.999 % tapentadol, in particular low, active doses, are preferred in order to avoid side effects. The medicament contains preferably 0.01 % by weight to 99.99 % by weight tapentadol, more preferably 0.1 to 90 % by weight, even more preferably 0.5 to 80 % by weight, most preferably 1.0 to 50 % by weight and in particular 5.0 to 20 % by weight. To avoid side effects, it may be advantageous at the start of the treatment to increase the amount of tapentadol to be administered gradually (titration) to allow the body to become accustomed to the active substance slowly. Preferably, tapentadol is first administered in a dose which is below the analgesically active dose.

Particularly preferably, the medicament has an oral pharmaceutical form, which is formulated for twice-daily administration and contains tapentadol in an amount of 20 to 260 mg as an equivalent dose based on the free base.

In another one of its embodiments, the present invention relates to tapentadol for use in a method for the treatment of irritable bowel syndrome.

In yet another one of its embodiments, the present invention relates to the use of tapentadol for the preparation of a medicament for the treatment of irritable bowel syndrome.

In yet another one of its embodiments, the present invention relates to a method for treating irritable bowel syndrome in a patient, preferably in a mammal, more preferably in a human, which comprises administering an effective and physiologically acceptable amount of tapentadol as described herein to a patient.

Preferably irritable bowel syndrome that includes irritable colon is selected from the group consisting of irritable bowel syndrome with diarrhoea, diarrhea-predominant irritable bowel syndrome, irritable bowel syndrome without diarrhoea, constipation-predominant irritable bowel syndrome, irritable bowel syndrome with alternating stool pattern (irritable bowel syndrome with alternating constipation and diarrhea, mixed irritable bowel syndrome) and post infectious irritable bowel syndrome. Particularly preferably the conditions to be addressed are irritable bowel syndrome with diarrhea and diarrhea-predominant irritable bowel syndrome.

Also preferably, irritable bowel syndrome is defined by ICD-10 (International Statistical Classification of Diseases and Related Health Problems, WHO edition, preferably version of 2007), i.e., it includes irritable colon [K58]. Preferably irritable bowel syndrome may include irritable bowel syndrome with diarrhoea [K58.0] and irritable bowel syndrome without diarrhea [K58.9]. Irritable bowel syndrome without diarrhoea may preferably also include irritable bowel syndrome not otherwise specified (NOS).

Even if the medicaments according to the invention exhibit few side effects only, it may be advantageous, for example, in order to avoid certain types of dependency to use morphine antagonists, in particular naloxone, naltrexone and/or levallorphan, in addition to tapentadol.

The present invention also relates to a kit comprising a medicament containing tapentadol (dosage forms) according to the invention.

The following gives a brief description of the figures:

**Figure 1** shows the effects of tapentadol hydrochloride on the twitch contractions of the isolated guinea pig ileum. Tapentadol hydrochloride was applied cumulatively to electrically stimulated guinea pig ileum preparations. After the last application of tapentadol hydrochloride, naloxone ($10^{-6}$ M) was added. Drug effects on the twitch reaction were calculated as percentage of pre-value and expressed as mean ± s.e.m.

**Figure 2** shows the anti-nociceptive effect of tapentadol hydrochloride in mustard oil colitis, as measured as inhibition of the spontaneous pain score.

**Figure 3** shows the anti-allodynic effect of tapentadol hydrochloride in mustard oil colitis, as measured as inhibition of the referred allodynia.

**Figure 4** shows the anti-hyperalgesic effect of tapentadol hydrochloride in mustard oil colitis, as measured as inhibition of the referred hyperalgesia.

**Figure 5** shows the time course of the anti-allodynic effect of curative tapentadol hydrochloride in mustard oil colitis, as measured as inhibition of the referred allodynia.

**Figure 6** shows the time course of the anti-hyperalgesic effect of tapentadol hydrochloride in mustard oil colitis, as measured as inhibition of the referred hyperalgesia.

In the respective figures Veh represents Vehicle solution: 0.9 % NaCl solution (Fresenius, Bad Homburg, FRG), PEG Veh represents Vehicle solution PEG 200 (Polyethylene glycol; molecular weight 200 g/mol).

The following examples serve for a further explanation of the invention but should not be construed as restrictive.

The studies presented below clearly show the inhibitory effects of tapentadol on ileum contractions and on visceral nociception, referred visceral hyperalgesia and allodynia. Thus, tapentadol addresses major symptoms of IBS, abnormal gastrointestinal (GI) motility and visceral hypersensitivity and referred pain.

**Examples:**

**1. Effects of tapentadol on the twitch contractions of the isolated guinea pig ileum.**

It was investigated whether tapentadol is able to modulate gastrointestinal motility. For this purpose, the responses to the compound were tested on electrically induced contractions of guinea pig ileum (so called Twitch reactions), which are known to be reduced e.g. by opioids (Paton, WDM. (1957) The action of morphine and related substances on contraction and on acetylcholine output of coaxially stimulated guinea-pig ileum. Br. J. Pharmacol. Chemother. 11: 119-127).

**1.1** *Experimental animals*

Male guinea pigs (PBW, Charles River, Kißlegg, FRG) weighing 250 - 350 g were used for the study. The animals were kept under standard housing conditions: light/dark rhythm (06.00 - 18.00 h light, 18.00 - 6.00 h dark); room temperature 22 $\pm$ 2°C, rel. air humidity 55 $\pm$ 5 %; 15 air changes per hour, air movement < 0,2 m/sec. The animals were given water and an exclusive diet of "Herilan RM 204" (Eggersmann Company, Rinteln/FRG) ad libitum. Before experimental preparation they were kept in groups of up to 5 animals in type IV Makrolon cages (Ebeco Company, Castrop-Rauxel, FRG). There were at least 4 days between delivery and test day.

**1.2** *Compounds*

Tapentadol hydrochloride was dissolved in aqua bidest. Final concentrations in the organ bath ranged from 3 · $10^{-8}$ to $10^{-5}$ M (cumulative drug application). As opioid antagonist naloxone ($10^{-6}$ M) was used.

**1.3** *Experimental method*

A four-compartment organ bath (Dept. Biotechnology, Grünenthal GmbH) was employed with 20 ml acrylic glass compartments, organ supports and force transducers (F10 Force transducers, Type 375, HSE, FRG) for the determination of isometric contractions. The organ baths were filled and emptied by means of a semi-automatic dosing arrangement. The nutrient medium in the organ baths was kept at room temperature. In a nutrient storage chamber and in the organ baths the nutrient solution was gassed with carbogen through a frit from 30 min before commencement of the experiment.

The nutrient solution had the following composition:

| | | |
|---|---|---|
| NaCl | 118.0 | mM |
| KCl | 4.8 | mM |
| $CaCl_2 \cdot 2H_2O$ | 1.3 | mM |
| $KH_2PO_4$ | 1.2 | mM |
| $MgSO_4 \cdot 7H_2O$ | 1.2 | mM |
| $NaHCO_3$ | 25.0 | mM |
| Glucose | 11,0 | mM |
| Ascorbic acid | 0.57 | mM |
| $Na_2$-EDTA | 0.03 | mM |
| (pH: 7.4 - 7.5) | | |

Parameter: contraction force [g]

**1.4** *Experimental performance*

Guinea pigs were killed in $CO_2$-atmosphere and the ileum was dissected free from adhering tissue, removed and suspended in the organ bath. After an incubation period of at least 30 min, transmural stimulation pulses were delivered with a duration of 1 ms and an amplitude of 180 mA at 0.03 Hz (Stimulator A310, WPI, FRG) and isometric contractions (Twitch reactions) were recorded. The preparations were pre-tensioned with 1 g. During the equilibration period of at least 30 min, the pre-tensioning was corrected to a constant level (approximately 1 g) and the nutrient was changed twice.

After registration of the pre-value, tapentadol hydrochloride was added to the organ bath in cumulative concentration steps as indicated. The exposition time for each cumulative concentration was 6 min. After the last application of the test compound, the opioid-antagonist naloxone ($10^{-6}$ M) was added without previous wash out of the test compound.

**1.5** *Evaluation*

Data were calculated as the mean stimulated contraction force during the period of 4 to 6 min. p. appl. and expressed as percentage of the pre-value. The mean contraction force in a period of 2 min before drug application was taken as pre-value. All results were expressed as means $\pm$ s.e.m. of $\geq 4$ single experiments. For determination of $IC_{50}$ values, regression lines (y = f log x) were constructed and $IC_{50}$ values with s.e.m. were calculated using a computer-assisted regression analysis program (Grünenthal GmbH). The reversal of the test compound's activity by the antagonist was expressed as the percentage of the effects of the combination of agonist and antagonist referred to the effects of the test compound alone in the highest concentration according to the following formula:

$$\% \text{ reversal} = 100\% - \frac{\text{max. effect}_{\text{test compound +antagonist}}}{\text{max. effect}_{\text{test compound}}} \times 100\%$$

with max. effect being:

100 % - % reduction of pre-value of twitch reaction at highest dose of test compound.

**1.6** *Results*

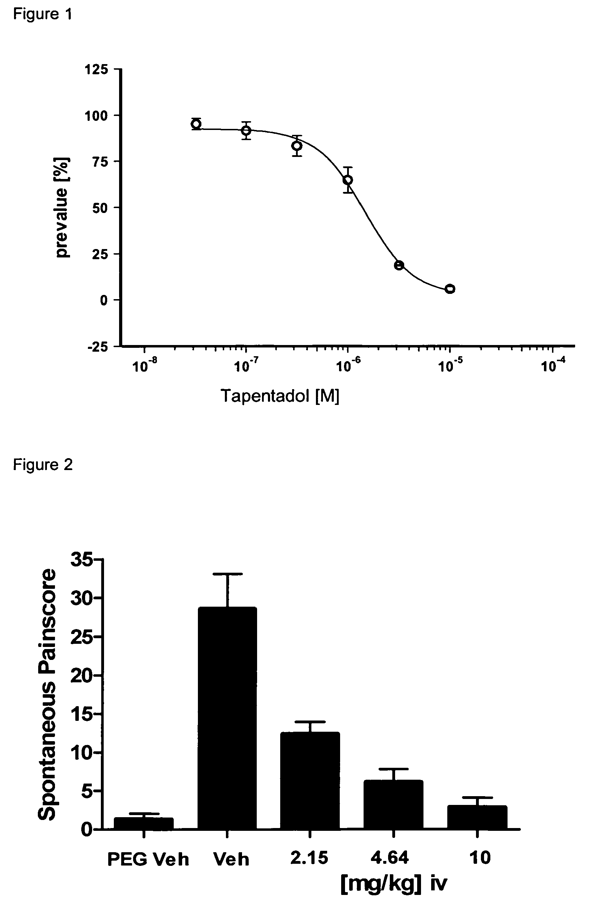

The compound reduced the electrically induced contractions of the isolated guinea pig ileum in a concentration dependent manner: The threshold concentration of the compound was about $10^{-7}$ M and an $IC_{50}$ value of 1.49 $\pm$ 0.20 $10^{-6}$ M was determined (see Figure 1 and Table 1). With increasing concentrations of the compound up to $10^{-5}$ M, the twitch reactions were almost blocked (reduction down to 5.77 $\pm$ 1.26 % of pre-value). The inhibitory effect of the compound was reversed by 33.3 % after addition of the opioid receptor antagonist naloxone ($10^{-6}$ M).

**Table 1:** Effects of the compound on Twitch contractions of the guinea pig ileum

|  | $IC_{50}$ [$10^{-6}$ M] | Max. effect [% pre-value] | % reversal by naloxone |
|---|---|---|---|
| tapentadol hydrochloride | 1.49 $\pm$ 0.20 | 5.77 $\pm$ 1.26 | 33.3 |

## 2. Effects of tapentadol on visceral hyperalgesia

The effects of tapentadol hydrochloride on visceral hyperalgesia were studied, which was induced by rectal administration of mustard oil in mice (according to Laird JM, Martinez-Caro L, Garcia-Nicas E, Cervero F. (2001) A new model of visceral pain and referred hyperalgesia in the mouse. Pain 92: 335-42). The typical visceral pain behaviour was quantified in three parameters: During the first minutes after mustard oil administration spontaneous visceral pain behaviour occurs. Following this period of spontaneous pain, referred allodynia and hyperalgesia can be quantified by means of von Frey filaments of different strength stimulating the abdomen of the mice.

**2.1** *Animals*

Male NMRI mice (28 - 38 g body weight) from a commercial breeder (Iffa Credo, France) were used. The animals were housed under standardized conditions: light/dark cycle (06.00 - 18.00 h light, 18.00 - 06.00 h dark), room temperature 20 - 24 °C, relative air humidity 35 - 70 %, 15 air changes per hour, air movement < 0,2 m/sec, tap water and standard diet ad libitum, macrolon type 4 cages with maximally 30 animals per cage. There were at least 5 days between delivery and start of the experiment.

**2.2** _Compounds_

Tapentadol hydrochloride was dissolved in vehicle solution and injected intravenously.

| | |
|---|---|
| Doses: | 2.15/ 4.64/ 10.0 mg/kg i.v. (prophylactic) |
| | 10.0/ 21.5/ 31.6 mg/kg i.v. (curative) |
| Administration volume: | 10 ml/kg |
| Vehicle solution: | 0.9 % NaCl solution (Fresenius, Bad Homburg, FRG) |

Mustard oil was dissolved in vehicle solution and administered intra-rectally
Dose: 50 $\mu$l of a 3.5 Vol-% solution per animal
Vehicle solution: PEG 200

**2.3** Experimental preparation

**2.3.1** _Induction of colitis_

Animals were habituated to the test conditions for 20 - 30 min and stimulated with von Frey filaments onto the abdominal wall. 10 stimulations with von Frey filaments of 1, 4, 8, 16, and 32 mN were applied in ascending order (i.e. 10 x 1 mN, 10 x 4 mN, etc.). Animals with more than 25 positive reactions during this phase were excluded. Vaseline was applied in the perianal area to avoid the stimulation of somatic areas with the irritant chemical. Colitis was induced by rectal administration of 50 $\mu$l mustard oil (3.5 %). Control animals were treated with vehicle (50 $\mu$l PEG200). Group sizes were n = 7 for all experiments.

**2.3.2** _Prophylactic treatment_

Tapentadol hydrochloride or vehicle was given intravenously (i.v.) 5 min before mustard oil. Seven animals were tested per group. The following parameters were counted:

2-12 min after mustard oil:

1. Spontaneous pain score: counting and scoring of visceral pain behaviours (Score 1 - 2, 1 = licking of abdominal wall, 2 = stretching, squashing, mounting, backward-movement or contraction of the flank muscles).

20 - 40 min after mustard oil

2. Referred allodynia (number of reactions): counting of withdrawal reactions against 10 stimulations with a 1 mN von Frey filament.
3. Referred hyperalgesia (referred pain score): counting and scoring of withdrawal reactions against 10 stimulations with a 16 mN von Frey filament (Score 1 - 3, 1 = lifting of abdomen, licking, movement, 2 = extrusion or flinching of hind paws, slight jumping, strong licking, 3 = strong jumping, vocalisation).

**2.3.3** _Curative treatment_

Tapentadol hydrochloride or vehicle was given intravenously (i.v.) 4 hours after mustard oil. Seven animals were tested per group. The following parameters were counted 10 minutes before and at different time points after administration of the test compound or vehicle:

1. Referred allodynia (number of reactions): counting of withdrawal reactions against 10 stimulations with a 1 mN von Frey filament.

2. Referred hyperalgesia (referred pain score): counting and scoring of withdrawal reactions against 10 stimulations with a 16 mN von Frey filament (Score 1 - 3, 1 = lifting of abdomen, licking, movement, 2 = extrusion or flinching of hind paws, slight jumping, strong licking, 3 = strong jumping, vocalisation).

EP 2 588 093 B1

Percent of maximal possible effect (% MPE) was calculated for each animal based on the baseline taken 10 min before administration of compound or vehicle.

*2.4 Statistical Analysis*

Significance was calculated by paired T-test (* $0.05 \geq p > 0.01$; ** $0.01 \geq p > 0.001$; *** $p \leq 0.001$. $ED_{50}$ values and 95 % confidence intervals were calculated by linear regression.

**2.5** *Results*

*2.5.1 Mustard oil colitis, prophylactic treatment*

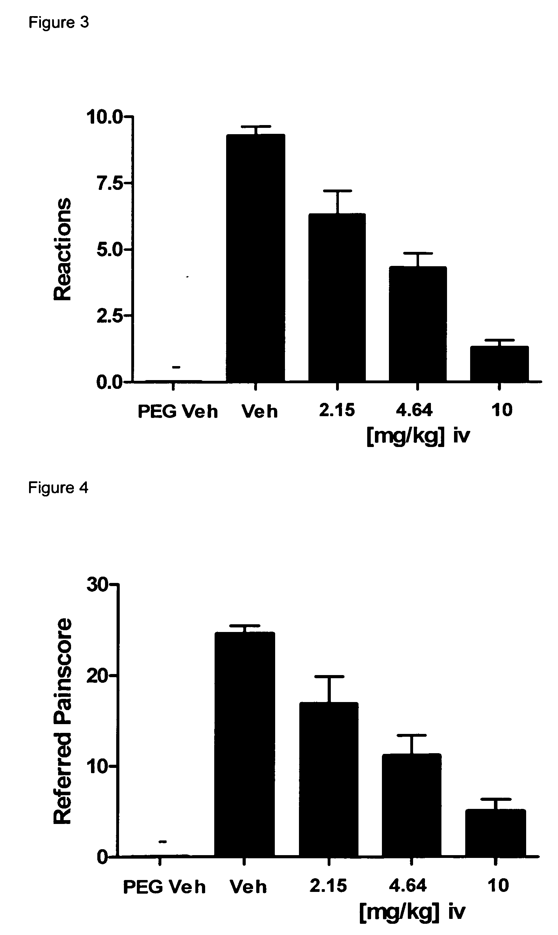

Tapentadol hydrochloride was tested in doses of 2.15/ 4.64 and 10.0 mg/kg i.v. It showed a dose dependent inhibition of all three visceral pain parameters. Spontaneous visceral pain behaviour (Figure 2), referred allodynia (Figure 3) and referred hyperalgesia (Figure 4) were inhibited with $ED_{50}$-values (95 % confidence intervals) of 1.47 (0.51 - 2.28)/ 3.75 (2.76 - 4.75)/ and 3.93 (2.32 - 5.69) mg/kg i.v., respectively.

*2.5.2 Mustard oil colitis, curative treatment*

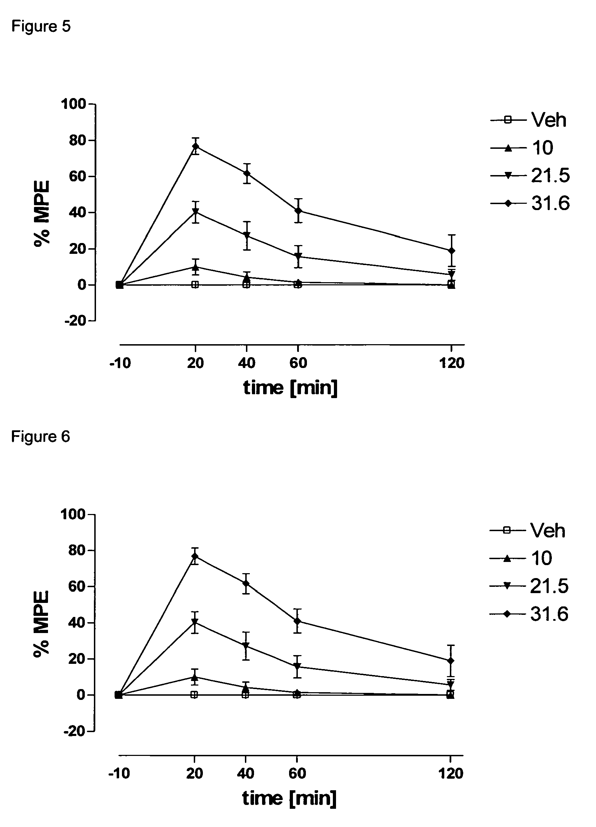

Curative administration of the compound in doses of 10.0/ 21.5 and 31.6 mg/kg i.v. after mustard oil showed a dose dependent inhibition of referred allodynia and referred hyperalgesia with $ED_{50}$-values (95 % confidence intervals) of 21.7 (19.3 - 25.0) and 16.3 (12.5 - 19.9) mg/kg i.v., respectively. The time course showed maximal efficacy 20 min after administration and duration of action of 1 to 2 h in the referred allodynia (Figure 5) and of more than 2 h in the referred hyperalgesia (Figure 6). Almost full efficacy was reached at a dose of 31.6 mg/kg, i.v.

**Claims**

1.   Tapentadol for use in the treatment of irritable bowel syndrome.

2.   Tapentadol for use according to claim 1, wherein irritable bowel syndrome is selected from the group consisting of irritable bowel syndrome with diarrhea, diarrhea-predominant irritable bowel syndrome, irritable bowel syndrome without diarrhea, constipation-predominant irritable bowel syndrome, irritable bowel syndrome with alternating stool pattern (irritable bowel syndrome with alternating constipation and diarrhea, mixed irritable bowel syndrome) and post infectious irritable bowel syndrome.

3.   Tapentadol for use according to claim 1 or 2, wherein it is present in a medicament.

4.   Tapentadol for use according to claim 3, wherein the medicament is solid.

5.   Tapentadol for use according to claim 3 or 4, wherein the medicament is formulated for oral administration.

6.   Tapentadol for use according to any one of claims 3-5, wherein the medicament is a tablet.

7.   Tapentadol for use according to any one of claims 3-6, wherein the medicament is formulated for administration twice daily (*bid*).

8.   Tapentadol for use according to any one of claims 3-7, wherein the medicament contains tapentadol in an amount of 10 to 300 mg.

**Patentansprüche**

1.   Tapentadol zur Verwendung in der Behandlung eines Reizdarmsyndroms.

2.   Tapentadol zur Verwendung gemäß Anspruch 1, wobei das Reizdarmsyndrom ausgewählt wird aus der Gruppe bestehend aus Reizdarmsyndrom mit Diarrhoe, Reizdarmsyndrom mit vorwiegender Diarrhoe, Reizdarmsyndrom ohne Diarrhoe, Reizdarmsyndrom mit vorwiegender Obstipation, Reizdarmsyndrom mit abwechselnden Stuhlmustern (Reizdarmsyndrom mit abwechselnder Obstipation und Diarrhoe, gemischtes Reizdarmsyndrom) und postinfektiöses Reizdarmsyndrom.

3. Tapentadol zur Verwendung gemäß Anspruch 1 oder 2, wobei es in einem Medikament vorliegt.

4. Tapentadol zur Verwendung gemäß Anspruch 3, wobei das Medikament fest ist.

5. Tapentadol zur Verwendung gemäß Anspruch 3 oder 4, wobei das Medikament für die orale Verabreichung formuliert ist.

6. Tapentadol zur Verwendung gemäß einem der Ansprüche 3-5, wobei das Medikament eine Tablette ist.

7. Tapentadol zur Verwendung gemäß einem der Ansprüche 3-6, wobei das Medikament für die zweimal tägliche Verabreichung formuliert ist.

8. Tapentadol zur Verwendung gemäß einem der Ansprüche 3-7, wobei das Medikament Tapentadol in einer Menge von 10-300 mg enthält.


**Revendications**

1. Tapentadol pour une utilisation dans le traitement du syndrome du côlon irritable.

2. Tapentadol pour une utilisation selon la revendication 1, le syndrome du côlon irritable étant sélectionné dans le groupe constitué du syndrome du côlon irritable avec diarrhée, du syndrome du côlon irritable avec prédominance de diarrhée, du syndrome du côlon irritable sans diarrhée, du syndrome du côlon irritable avec prédominance de constipation, du syndrome du côlon irritable avec alternance du type de selles (syndrome du côlon irritable avec alternance de constipation et de diarrhée, syndrome du côlon irritable mixte) et du syndrome du côlon irritable post-infection.

3. Tapentadol pour une utilisation selon la revendication 1 ou 2, celui-ci étant présent dans un médicament.

4. Tapentadol pour une utilisation selon la revendication 3, le médicament étant solide.

5. Tapentadol pour une utilisation selon la revendication 3 ou 4, le médicament étant formulé en vue d'une administration par voie orale.

6. Tapentadol pour une utilisation selon l'une quelconque des revendications 3 à 5, le médicament étant un comprimé.

7. Tapentadol pour une utilisation selon l'une quelconque des revendications 3 à 6, le médicament étant formulé en vue d'une administration deux fois par jour (*b.i.d.*).

8. Tapentadol pour une utilisation selon l'une quelconque des revendications 3 à 7, le médicament contenant le tapentadol dans une quantité de 10 à 300 mg.

Figure 1

Figure 2

Figure 3

Figure 4

Figure 5

Figure 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009094563 A2
- EP 1997493 A1
- EP 1985292 A1
- EP 693475 A
- US 200558706 A

**Non-patent literature cited in the description**

- **POSSERUD I ; ERSRYD A ; SIMRÉN M.** Functional findings in irritable bowel syndrome. *World J. Gastroenterol.,* 2006, vol. 12 (18), 2830-2838
- **GRUNDMANN O ; YOON SL.** Irritable bowel syndrome: epidemiology, diagnosis and treatment: an update for health-care practitioners. *J. Gastroenterol. Hepatol.,* 2010, vol. 25 (4), 691-699
- *CHEMICAL ABSTRACTS,* 175591-23-8
- Remington's Pharmaceutical Sciences. Mack Publishing Company, 1985
- **R. SPILLER.** probiotics and prebiotics in irritable bowel syndrome. *Aliment Pharmacol Ther,* vol. 28, 385-396
- **TM TZSCHENTKE et al.** *Drugs of the Future,* 2006, vol. 31 (12), 1053
- International Statistical Classification of Diseases and Related Health Problems. 2007
- **PATON, WDM.** The action of morphine and related substances on contraction and on acetylcholine output of coaxially stimulated guinea-pig ileum. *Br. J. Pharmacol. Chemother.,* 1957, vol. 11, 119-127
- **LAIRD JM ; MARTINEZ-CARO L ; GARCIA-NICAS E ; CERVERO F.** A new model of visceral pain and referred hyperalgesia in the mouse. *Pain,* 2001, vol. 92, 335-42